# EUROPEAN PATENT APPLICATION

(11) **EP 1 997 485 A1**
(43) Date of publication of application: **03.12.2008**
(21) Application number: 07708206.3
(22) Date of filing: 08.02.2007
(51) Int. Cl.: A61K 31/192, A61K 31/198, A61P 1/16, A61P 43/00

(54) **PHARMACEUTICAL COMPOSITION COMPRISING MEGLITINIDE FOR PREVENTION OF HEPATIC FIBROSIS**

(30) Priority: 08.02.2006 JP 2006031269
(71) Applicant: Kurume University, Kurume-shi Fukuoka 8300011 (JP); Ajinomoto Co., Inc., Tokyo 104-8315 (JP)
(72) Inventor: UENO, Takato, Kurume-shi, Fukuoka 830-0011 (JP); MORITA, Yasuyo, Kurume-shi, Fukuoka 830-0011 (JP); SATA, Michio, Kurume-shi, Fukuoka 830-0011 (JP); OKANO, Akira, Kawasaki-shi, Kanagawa 210-8681 (JP)
(74) Representative: Nicholls, Kathryn Margaret
(86) International application number: PCT/JP2007/052182
(87) International publication number: WO 2007/091623

(57) **Abstract**

The present invention discloses pharmaceutical compositions for preventing, improving or treating hepatic fibrosis, hepatocellular degeneration or cirrhosis, which contain a meglitinide compound such as nateglinide.

## Description

### Technical Field of the Invention

The present invention relates to pharmaceutical compositions for preventing, improving or treating hepatic fibrosis, hepatocellular degeneration or cirrhosis, which contain a drug(s) corresponding to a meglitinide compound. More specifically, the present invention relates to pharmaceutical compositions for preventing, improving or treating hepatic fibrosis, hepatocellular degeneration or cirrhosis, which contain nateglinide.

### Background of the Invention

In most hepatic diseases such as virus hepatitis, alcoholic hepatitis and autoimmune hepatitis, cirrhosis which is the final stage of hepatic fibrosis induces life-threatening diseases, such as hepatocellular cancer, esophagus and gastric varices. Therefore, it is urgently needed to establish the method for suppressing hepatic fibrosis including cirrhosis. It is reported that, in virus hepatitis, the virus removal therapy with interferons and antivirus agents has an improvement effect on hepatic fibrosis. However, the complete removal ratio of the virus of hepatitis C, which is one of the representative hepatic diseases, is around 60% in Japan though the most recent interferons and antivirus agents are used in combination. Thus, there is urgent need to establish the therapeutics preventing the progression to cirrhosis in such non-responders patients. Meanwhile, recent years, based on prevalence of lifestyle-related diseases, nonalcoholic steatohepatitis (NASH) has been acknowledged as a problem as the similar hepatic pathology to alcoholic hepatitis in the absence of excessive alcohol ingestion and hepatitis virus infections. Matteoni, et al. reported that based on the followed up study among different type of non alcoholic fatty liver disease, such as simple fatty livers; simple fatty livers with inflammation; simple fatty livers with hepatocellular degeneration; and simple fatty livers with Mallory body or fibrosis 20% of those with hepatocellular degeneration and those with Mallory body or fibrosis developed cirrhosis (Non-patent Literature 1). Simple fatty livers with hepatocellular degeneration and simple fatty livers with Mallory body or fibrosis are categorized as NASH. Further, the long-term follow-up of patients diagnosed as NASH in the cirrhosis stage revealed that 20-30% of those developed liver cancer (Non-patent Literatures 2 and 3). It is assumed that the number of patients with NASH having possibilities to develop cirrhosis or liver cancer is 5.6 million in US and 1.5 million in Japan based on the prevalence of western style diets and increase in obesity. (Non-patent Literature 4), and, therefore, effective treatments are needed. Though the pathogenic mechanism of NASH is not clarified, Day et al. have advocated 2 hit theory (Non-patent Literature 5). Namely, as the 1st hit, fatty liver occurs due to the disorders of glucose metabolism and lipid metabolism. Then, as the 2nd hit, inflammation or the like increases oxidative stress, and hepatic fibrosis or cirrhosis occurs via hepatocellular degeneration or apoptosis. Since it has been thought that fatty liver recognized as 1^{st} hit is induced by lifestyle-related diseases, especially disorders of glucose metabolism such as insulin resistance, impaired glucose tolerance, postprandial hyperglycemia and diabetes, anti-diabetic agents are supposed to be. applicable. There are reports on metformin (Non-patent Literature 6) and glitazones (Non-patent Literatures 7 and 8) which lower blood glucose level by improving insulin resistance. However, they are not satisfactory as therapeutic agents for NASH because the effect of metformin is limited and it is reported that glitazones increase the body weight and have concern about hepatic dysfunction. Besides, sulfonylureas (SU agents) and insulin (Non-patent Literature 9) are difficult to adapt to NASH since it is reported that they induce fatty liver which is a risk factor for NASH by continuously increasing insulin concentration. Under such circumstances, Kitahara et al. found that fast-acting insulin secretagogues including nateglinide lower insulin concentration and blood glucose concentration in the portal vein, and, as a result, they inhibit fatty acid synthesis in the liver and the amount of liver TG, and diminish fatty liver which is a risk for NASH. Namely, in Patent Literature 1, they found that the administration of nateglinide before glucose load inhibits glucose⁻ induced expression of ATP-citrate lyase, which is a rate-controlling enzyme in fatty acid synthesis, in the liver of a diabetic model animal. Further, they also found that, in clinical practice, twelve-week administration of nateglinide statistically significantly inhibits GOT and GPT, each of which is recognized as the index of hepatic injury, in 53 fatty liver cases of those with diabetes or 5 cases where GPT>GOT among fatty livers of those with diabetes. Thus, they indicate that improvement in fatty liver also inhibits hepatic disorders which are the subsequent step thereof and have completed the invention. After that, when nateglinide was administered for 16 weeks to 5 cases of patients with diabetes who were definitely diagnosed as NASH by a liver biopsy, it was confirmed by liver biopsies before and after the administration that the fatty liver diminished, and hepatic inflammation, hepatic degeneration or hepatic fibrosis was improved (Non-patent Literature 10). It was thought to be sufficiently explainable that the expression of the medicinal effect relating to the diminishment of fatty liver and the improvements in hepatic inflammation, hepatic degeneration and hepatic fibrosis was the result of an improvement effect on fatty liver which occurred as a result of changes of insulin secretion pattern by fast-acting insulin secretagogues. Besides, it has not been reported at all until now that nateglinide improves hepatic inflammation, hepatic degeneration or hepatic fibrosis in the action mechanism other than this, for example, in the action without insulin secretion. At present, there is no therapeutic agent for hepatic fibrosis which is available in clinical experts.

Meanwhile, the degree of hepatic fibrosis herein mentioned is evaluated by the steps comprising of treating the tissue section of liver biopsy samples with the method of specifically staining the fibrillary element such as Masson's trichrome stain, Sweet's reticulin stain or Azan stain; and then determining the quantity thereof per distinctive fibrosis images or evaluating them as stages. As one example, since hepatocellular necrosis or degeneration in NASH often occurs in areas of zone 3, the portal fibrosis is evaluated corresponding to the degree thereof on a scale of 0 to 4 when pericellular fibrosis, perivenular fibrosis or pathology of the region corresponding to the areas of zone 3 is advanced. Further, the degree is comprehensively classified as: Stage 1: perivascular/perisinusoldal/pericellular fibrosis partially or widely exists centering on zone 3; Stage 2: portal fibrosis partially or widely exists in addition to Stage 1; Stage 3: bridging fibrosis partially or widely exists; and Stage 4: cirrhosis.

Hepatocellular degeneration is determined as ballooning of hepatocytes after treating the section of liver biopsy tissues with hematoxylin-eosin staining. Hepatocellular degeneration is evaluated based on the number of the cells observed in the visual field (Non-patent Literature 11).
Patent Literature 1: WO 03/099332
Non-patent Literature 1: Gastroenterology 116 1413-1419, 1999
Non-patent Literature 2: Shimada M et al. Journal of Hepatology 37 154-160, 2002
Non-patent Literature 3: Ratziu V et al. Hepatology 35 1485-1493, 2002
Non-patent Literature 4: Wanless IR et al. Hepatology 12 1106-1110 1990
Non-patent Literature 5: Day CP et al. Gastroenterology 114 842-845. 1998
Non-patent Literature 6: Marchesini G et al. Lancet 358 893-894 2001
Non-patent Literature 7: Promrat K et al. Hepatology 39 188-196 2004
Non-patent Literature 8: Neuschwander-Tetri BA et al. Hepatology 38 1008-1017 2003
Non-patent Literature 9: Khalili K et al. AJR Am J Roentgenol. 180 1601-1604 2003
Non-patent Literature 10: Morita Y et al. Hepato-Gastorenterology 52 1338-1343, 2005
Non-patent Literature 11: Brunt EM et al. Am J Gastroenterol. 1999 94: 2467-2474

### Disclosure of the Invention

The object of the present invention is to provide a pharmaceutical composition for preventing, improving or treating hepatic fibrosis.

The further object of the present invention is to provide a pharmaceutical composition for preventing, improving or treating hepatocellular degeneration.

The yet further object of the present invention is to provide a pharmaceutical composition for preventing, improving or treating cirrhosis.

The inventors investigated the relationship between the improvement effect on diabetes and the improvement effect on hepatic inflammation, degeneration or fibrosis of each case by administering nateglinide to patients with both diabetes and NASH, and found that, surprisingly, even in the cases where symptoms of diabetes were not improved, hepatic inflammation, degeneration or fibrosis thereof was improved. The present invention has been completed based on these findings. The cases of NASH include a case where disorder of glucose metabolism is not seen; a case where insulin resistance is seen; a case where impaired glucose tolerance is seen; and early, medium and end stage diabetes. Until now, it has been supposed that nateglinide applies only to impaired glucose tolerance, a delayed secretion of insulin, postprandial hyperglycemia and early stage diabetes. However, the findings of the present invention show that nateglinide can apply to cases where the diabetic indexes are not improved by fast-acting insulin secretagogues such as nateglinide and the treatment of NASH of patients with end stage diabetes whose insulin can not be effectively secreted by the pancreas. Further, the findings of the present invention show that low-concentrated nateglinide, which does not induce the fast-acting insulin secretion, directly acts on the liver to have the possibilities of inhibiting, preventing, improving or treating hepatic inflammation, degeneration or fibrosis of all patients including those having high insulin resistance and those with end stage diabetes whose insulin can not be effectively secreted by the pancreas.

Namely, the present invention provides a pharmaceutical composition for preventing, improving or treating hepatic fibrosis which contains a meglitinide compound, preferably nateglinide.

The present invention also provides a pharmaceutical composition for preventing, improving or treating hepatic degeneration which contains a meglitinide compound, preferably nateglinide.

The present invention further provides a pharmaceutical composition for preventing, improving or treating cirrhosis which contains a meglitinide compound, preferably nateglinide.

The present invention yet further provides use of a meglitinide compound, preferably nateglinide, for the preparation of a pharmaceutical composition for preventing, improving or treating hepatic fibrosis, hepatic degeneration or cirrhosis.

According to the present invention, it is possible to provide pharmaceutical compositions useful for preventing, improving or treating hepatic fibrosis, hepatic degeneration or cirrhosis. As the meglitinide compound, nateglinide is particularly useful.

### Best Mode for Carrying out the Invention

In the specification, the meglitinide compound is a compound which is structurally similar to meglitinide as described in Hormone and Metabolic Research, vol. 27, pp. 263-266, 1995. Preferable examples of the meglitinide compound include those of the following formulae, that is, D-phenylalanine derivatives such as (-)-N-(trans-4-isopropylcyclohexanecarbonyl)-D-phenylalanine (referred to as "nateghnide"); benzylsuccinic acid derivatives such as (2S)-2-benzyl-4-[(3aR,7aS)-octahydro-2H-isoindole-2-yl]-4-oxobutanic acid (referred to as "had-1229"); and benzoic acid derivatives such as (S)-2-ethoxy-4-{2-[[3-methyl-1-[2-(1-piperidinyl) phenyl]butyl]amino]-2-oxoethyl} benzoic acid (referred to as "repaglinide"). Meglinitide, nateglinide, KAD-1229 and repaglinide are more preferable.

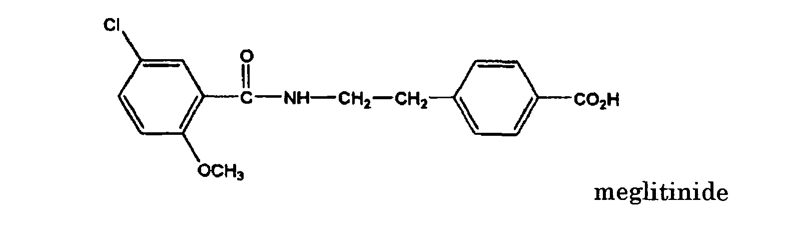

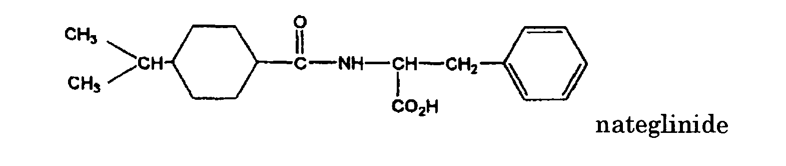

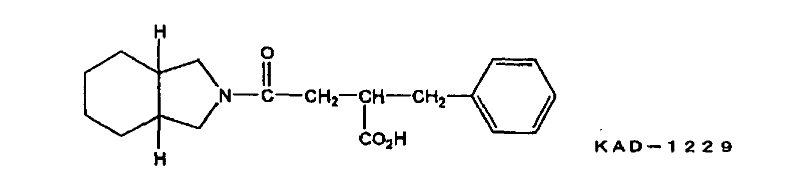

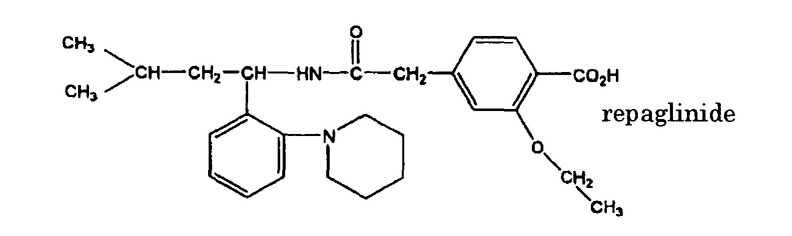

In the present invention, the above meglitinide compound can be used alone or in combination with at least one other agents selected from hypoglycemic agents, therapeutic agents for hyperlipidemia, antihypertensive agents, antioxidant agents, liver supporting agents and anti-inflammatory agents. Examples of hypoglycemic agents include insulin derivatives such as insulin, lispro and glargine; sulfonylurea agents such as tolubutamide, gliclazide, glibenclamide and glimepiride; α -glucosidase inhibitors such as acarbose, voglibose and miglitol; biguanides such as metformin and phenformin; thiazolidines such as pioglitazone, rosiglitazone and toroglitazone or PPAR γ agonists of nonthiazolidine scaffold such as GI-262570, JTT-501, YM-440, NN-622 and KRP-297; insulin sensitizers containing an antagonist(s); adrenaline β 3 receptor agonists such as AJ-9677; insulin-like agonists such as CLX-0901; GLP-1 agonists such as GLP-1, Exendin-4 and NN-2211; DPPIV inhibitors such as DPP-728A; SGLT inhibitors such as T-1095; and ACC inhibitors.

Examples of therapeutic agents for hyperlipidemia which decrease lipids in the blood include HMG-CoA reductase inhibitors such as pravastatin, simvastatin, fluvastatin, cerivastatin, atorvastatin and itavastatin; fibrates such as simfibrate, clofibrate, clinofibrate, bezafibrate and fenofibrate; anion-exchange resins such as colestimide and cholestiramine; and nicotinic acid preparations such as nicomol and niceritrol.

Examples of antihypertensive agents include angiotensin-convertase inhibitors, calcium channel antagonists and angiotensin receptor blockers.

Examples of antioxidant agents include vitamins such as vitamin C and vitamin E, N-acetyl cysteine, probucol, eicosapentaenoic acid, and esters thereof.

Examples of liver supporting agents include ursodeoxycholic acid and betaine. Further, examples of anti-inflammatory agents include cytokine production blockers such as pentoxyphylline and anti-TNF antibodies.

The effect of the present invention can be further enhanced by using the above agents in combination. The combination thereof may be a single dosage form wherein both the meglitinide compound and the other agents are contained together, or two different dosage forms administered simultaneously or at intervals wherein the meglitinide compound and the other agents are contained separately.

In the pharmaceutical composition, each amount of the meglitinide compound and at least one other agents selected from the group consisting of hypoglycemic agents, therapeutic agents for hyperlipidemia, antihypertensive agents, antioxidant agents, liver supporting agents and anti-inflammatory agents can be appropriately determined, and the content of the meglitinide compound is preferably 0.1 to 99 % by weight. Further, at least one other agents selected from the group consisting of hypoglycemic agents, therapeutic agents for hyperlipidemia, antihypertensive agents, antioxidant agents, liver supporting agents and anti-inflammatory agents is preferably 1 to 99.9 % by weight.

Besides, in addition to the above agents, the pharmaceutical compositions of the present invention can contain various pharmacologically acceptable substances for preparation (as formulation additive) (herein after also referred to as "pharmaceutically acceptable carriers"). The substances for preparation can be appropriately selected corresponding to the dosage form of the preparation, and examples thereof include excipients, diluents, additives, disintegrating agents, binders, coating agents, lubricant agents, gliding agents, lubricants, flavoring agents, sweetening agents and solubilizing agents. Further, more specific examples of the substances for preparation include magnesium carbonate, titanium dioxide, lactose, mannitol and other sugars, talc, milk protein, gelatin, starch, cellulose and derivatives thereof, animal and vegetable oil, polyethylene glycol and solvents such as sterilized water and monohydroxy alcohol or polyalcohol, e.g. glycerol.

The pharmaceutical compositions of the present invention are especially useful in preventing, improving or treating hepatic fibrosis or hepatic degeneration of the patients with diabetes whose diabetes is not improved by the administration of the meglitinide compound, and also useful in inhibiting transformation into cirrhosis.

The pharmaceutical preparations of the present invention can be formulated into the various administration forms such as oral, intraperitoneal, transdermal and inhalation administration forms. Examples thereof include suitable solid or liquid preparation forms such as granules, powder, coated tablets, tablets, (micro)capsules, suppositories, syrup, juice, suspension, emulsion, drops, solutions for injection and preparations which prolong the release of the active substance.

The dose of the meglitinide compound (the active ingredient) used as the compositions of the present invention can be suitably selected, depending on the kind of the meglitinide compound, the kind of the complications, degree of the symptoms of complications or nervous disorders, the form of the preparations, with or without side effects and the degree thereof. For example, in the case of the preparation containing nateglinide as the active ingredient, it can be orally administered to the patient preferably in the amount of around 10mg to 10g in net weight of nateglinide per day, more preferably in the amount of around 30mg to 1g, and particularly preferably around 90 to 270mg. It is possible to further increase the amount in a serious case. As for the number of doses and timing of the administration, it is possible to administer the agent of the present invention once in a few days or once per day. Usually, it is administered several times per day, for example, twice to four times, preferably before eating. In the case of the parenteral administration such as the intravenous administration, the administered dose may be around 1/10 to 1/20 of that of the oral administration.

In case where the meglitinide compound is mixed or combined with at least one agents selected from hypoglycemic agents, therapeutic agents for hyperlipidemia, antihypertensive agents, antioxidant agents, liver supporting agents or anti-inflammatory agents, the content in the composition or dose of the other agents can be appropriately determined based on the suitable effective dose of each agents, which has already developed, or which is known, e.g., under development.

### Example

5 patients were selected as the subjects, who had clinical features of fatty liver found by the abdominal ultrasound or the abdominal CT, had negative test results in various virus markers and autoantibody, could deny a drinking history or a drug history; were diagnosed as NASH by a liver biopsy based on Brunt's scale (Am J Gastroenterol. 1999 94: 2467-2474), and had diabetes on which diet and exercise therapies could not sufficiently produce the therapeutic effect. Meanwhile, the examination protocol passed the ethical review of the clinical establishment, and obtained informed consent from all subjects. 90mg of nateglinide was orally administered to these 5 patients three times per day before each meal for 16 weeks. In 0th week and 12th week or 16 week, as the diabetic indexes, the fasting blood glucose, the blood glucose 2 hours after the glucose tolerance test, and hemoglobin A1c, as the evaluation of the liver, the degree of fatty liver determined by liver/spleen ratio of CT and liver biopsy tissues for each case were histologically analyzed to score hepatocellular degeneration, pericellular fibrosis, perivenular fibrosis, portal fibrosis or the like (Brunt EM et al. Am J Gastroenterol. 1999 94: 2467-2474).

Table 1 shows the indexes before the administration of nateglinide.

Table 2 shows the indexes after the administration of nateglinide.

Table 3 shows the variations Δ (value before the admin. - value after the admin.) of each index of 0th and 12th weeks.

**Table 1 Measurement indexes before the administration of nateglinide**

| | HbA1c (%) | fasting blood glucose (mg/dl) | BG 2 hrs.^{*1} (mg/dl) | stage | pericellular fibrosis | portal fibrosis | perivenular fibrosis | grade | hepatocellular degeneration | portal vein inflammation | portal area neutrophil infiltration | CT score liver/spleen ratio |
|---|---|---|---|---|---|---|---|---|---|---|---|---|
| Case 1 | 7.2 | 183 | 354 | 3 | 3 | 3 | 3 | 3 | 3 | 2 | 1 | 0.71 |
| Case 2 | 4.9 | 94 | 207 | 1 | 1 | 1 | 2 | 2 | 2 | 1 | 1 | 0.715 |
| Case 3 | 5.6 | 101 | 229 | 3 | 2 | 3 | 2 | 2 | 2 | 1 | 1 | 0.94 |
| Case 4 | 6.1 | 126 | 193 | 1 | 2 | 1 | 2 | 1 | 1 | 1 | 0 | 0.7 |
| Case 5 | 7 | 154 | 286 | 2 | 3 | 2 | 2 | 3 | 3 | 2 | 1 | 0.7 |

**Table 2 Measurement indexes after the administration of nateglinide**

| | HbA1c (%) | fasting blood glucose (mg/dl) | BG 2 hrs.^{*1} (mg/dl) | stage | pericellular fibrosis | portal fibrosis | perivenular fibrosis | grade | hepatocellular degeneration | portal vein inflammation | portal area neutrophil infiltration | CT score liver/spleen ratio |
|---|---|---|---|---|---|---|---|---|---|---|---|---|
| Case 1 | 7.1 | 163 | 337 | 2 | 1 | 2 | 2 | 2 | 2 | 1 | 1 | 0.8 |
| Case 2 | 5 | 100 | 118 | 1 | 0 | 0.5 | 1 | 1.5 | 1 | 0 | 1 | 1.05 |
| Case 3 | 5.5 | 106 | 199 | 2 | 1 | 2 | 1 | 1 | 1 | 0 | 0 | 1 |
| Case 4 | 5.6 | 119 | 105 | 1 | 1 | 1 | 1 | 1 | 1 | 0 | 0 | 0.9 |
| Case 5 | 6.2 | 115 | 238 | 1.5 | 1 | 1.5 | 1 | 2 | 1 | 0 | 0 | 0.95 |

**Table 3 Variarion of measurement indexes before and after the administration**

| 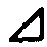 : value before admin. - value after admin. | | | | | | | | | | | | |
|---|---|---|---|---|---|---|---|---|---|---|---|---|
| | 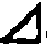 HbA1c (%) | 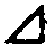 fasting blood glucose (mg/dl) | 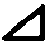 BG 2 hrs.^{*1} (mg/dl) | 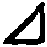 stage | 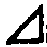 pericellular fibrosis | 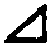 portal fibrosis | 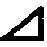 perivenular fibrosis | 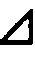 grade | 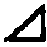 hepatocellular degeneration | 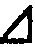 portal vein inflammation, | 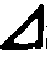 portal area neutrophil infiltration | 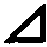 CT score liver/spleen ratio |
| Case 1 | 0.1 | 20 | 17 | 1 | 2 | 1 | 1 | 1 | 1 | 1 | 0 | -0.09 |
| Case 2 | -0.1 | -6 | 89 | 0 | 1 | 0.5 | 1 | 0.5 | 1 | 1 | 0 | -0.335 |
| Case 3 | 0.1 | -5 | 30 | 1 | 1 | 1 | 1 | 1 | 1 | 1 | 1 | -0.06 |
| Case 4 | 0.5 | 7 | 88 | 0 | 1 | 0 | 1 | 0 | 0 | 1 | 0 | -0.2 |
| Case 5 | 0.8 | 39 | 48 | 0.5 | 2 | 0.5 | 1 | 1 | 2 | 2 | 1 | -0.25 |

| | | | | | | | | | | | | |
|---|---|---|---|---|---|---|---|---|---|---|---|---|
| *1 "BG 2 hours." represents blood glucose 2 hours after the glucose tolerance test | | | | | | | | | | | | |

In the cases whose diabetic indexes were improved by the administration of nateglinide, improvements were also observed in fatty liver, hepatocellular degeneration, pericellular fibrosis, perivenular fibrosis or portal fibrosis. In the cases whose diabetic indexes were not improved, an improvement effect on fatty liver was low. From the observations, it can be seen that the improvement effect on fatty liver might correlate with the improvement effect on diabetes. On the other hand, even in these cases whose diabetic indexes were not improved (Cases 1 and 3), almost the same improvements were observed in hepatocellular degeneration, pericellular fibrosis, perivenular fibrosis or portal fibrosis as those in the cases whose diabetic indexes were improved.

## Claims

1. A pharmaceutical composition for preventing, improving or treating hepatic fibrosis which contains a meglitinide compound.

2. The pharmaceutical composition according to claim 1, wherein the meglitinide compound is selected from the group consisting of meglitinide, nateglinide, KAD-1229 and repaglinide.

3. The pharmaceutical composition according to claim 2, wherein the meglitinide compound is nateglinide.

4. The pharmaceutical composition according to any one of claims 1 to 3, which is administered to a patient with diabetes.

5. The pharmaceutical composition according to claim 4, which is administered to a patient with diabetes whose diabetes is not improved by the administration of the meglitinide compound.

6. Use of a meglitinide compound for the preparation of a pharmaceutical composition for preventing, improving or treating hepatic fibrosis.

7. A pharmaceutical composition for preventing, improving or treating hepatocellular degeneration which contains a meglitinide compound.

8. The pharmaceutical composition according to claim 7, wherein the meglitinide compound is selected from the group consisting of meglinitide, nateglinide, KAD-1229 and repaglinide.

9. The pharmaceutical composition according to claim 8, wherein the meglitinide compound is nateglinide.

10. The pharmaceutical composition according to any one of claims 7 to 9, which is administered to a patient with diabetes.

11. The pharmaceutical composition according to claim 10, which is administered to a patient with diabetes whose diabetes is not improved by the administration of the meglitinide compound.

12. Use of a meglitinide compound for the preparation of a pharmaceutical composition for preventing, improving or treating hepatocellular degeneration.

13. A pharmaceutical composition for preventing, improving or treating cirrhosis which contains a meglitinide compound.

14. The pharmaceutical composition according to claim 13, wherein the meglitinide compound is selected from the group consisting of meglitinide, nateglinide, KAD-1229 and repaglinide.

15. The pharmaceutical composition according to claim 14, wherein the meglitinide compound is nateglinide.

16. The pharmaceutical composition according to any one of claims 13 to 15, which is administered to a patient with diabetes.

17. The pharmaceutical composition according to claim 16, which is administered to a patient with diabetes whose diabetes is not improved by the administration of the meglitinide compound.

18. Use of a meglitinide compound for the preparation of a pharmaceutical composition for preventing, improving or treating cirrhosis.
